# EUROPÄISCHE PATENTSCHRIFT

(11) **EP 3 074 372 B1**
(45) Veröffentlichungstag und Bekanntmachung des Hinweises auf die Patenterteilung: **14.06.2017**
(21) Anmeldenummer: 14799491.7
(22) Anmeldetag: 19.11.2014
(51) Int. Cl.: C07C 67/10, C07C 69/24

(54) **VERFAHREN ZUR RUTHENIUM-KATALYSIERTEN UMVINYLIERUNG VON CARBONSÄUREN**
PROCESS FOR THE RUTHENIUM-CATALYZED TRANSVINYLATION OF CARBOXYLIC ACIDS
PROCÉDÉ POUR LA TRANSVINYLATION D'ACIDES CARBOXYLIQUES CATALYSÉE PAR LE RUTHÉNIUM

(30) Priorität: 29.11.2013 DE 102013224496
(43) Veröffentlichungstag der Anmeldung: 05.10.2016
(73) Patentinhaber: Wacker Chemie AG, 81737 München (DE)
(72) Erfinder: GIGLER, Peter, 85221 Dachau (DE); STOHRER, Jürgen, 82049 Pullach (DE)
(74) Vertreter: Schuderer, Michael
(86) Internationale Anmeldenummer: PCT/EP2014/075014
(87) Internationale Veröffentlichungsnummer: WO 2015/078747

(56) Entgegenhaltungen:
- EP-A2- 0 351 603
- WO-A1-2013/117294

## Beschreibung

Die Erfindung betrifft ein Verfahren zur selektiven Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren.

Die Umvinylierung von Carbonsäuren dient der Darstellung von Vinylestern. Darunter versteht man die Übertragung einer Vinyl-Einheit eines Eduktvinylesters (1V) auf eine Eduktcarbonsäure (2S) unter Generierung eines Produktvinylesters (2V) und der korrespondierenden Säure des Eduktvinylesters (1S).

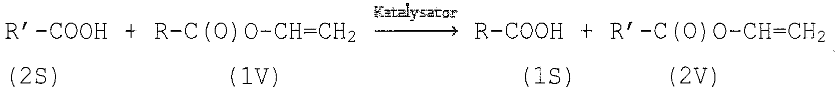

Aus der EP 376075 B1 ist die Umvinylierung von Vinylestern mit Carbonsäuren in Gegenwart von Palladiumkatalysator bekannt, wobei Kupferbromid und spezielle Lithiumverbindungen als Cokatalysatoren eingesetzt werden.

Neben Palladium-Katalysatoren und Quecksilber-Katalysatoren werden im Stand der Technik zur Umvinylierung von Vinylestern mit Carbonsäuren auch Ruthenium-Verbindungen als Katalysator eingesetzt. Ruthenium-Verbindungen zeichnen sich durch ihre hohe Löslichkeit, geringe Flüchtigkeit und hohe thermische Stabilität aus. Hinzu kommt eine hohe, Temperatur-induzierbare Aktivität. Problematisch beim Einsatz von Ruthenium-Verbindungen als Katalysatoren bei der Umvinylierung von Vinylestern mit Carbonsäuren ist allerdings die Carbonsäureanhydrid-Bildung als Nebenreaktion zur Umvinylierung. Diese Nebenreaktion reduziert die Selektivität der Reaktion. Zusätzlich handelt es sich bei den Anhydriden der Eduktcarbonsäure um relativ hoch siedende Nebenprodukte, welche sich nur mit erheblichem Aufwand von der katalysator-haltigen Reaktionsmasse abtrennen lassen und bei deren Wiedereinsatz somit akkumuliert würden.

In der EP 351603 A2 (US4981973) wird ein Verfahren zur Umvinylierung von Carbonsäuren unter Verwendung verschiedener Ru-Verbindungen als Katalysator beschrieben. Zur Verschiebung des Gleichgewichts wird empfohlen, eines der Reaktionsprodukte kontinuierlich aus dem Reaktionsgemisch zu entfernen. Bei Anwesenheit von Wasser wird die Erhöhung der Katalysatorkonzentration empfohlen. So wird in Beispiel 60 bei einem Wassergehalt von 2 % eine zehnfach höhere Katalysatorkonzentration als im wasserfreien Fall eingesetzt. Nach Abschluss der Umvinylierung wird das Produktgemisch destillativ aufgetrennt. Die Rückführung des Ru-Katalysators wird nicht beschrieben.

Die EP 497340 A2 (US5210207) beschreibt ein Umvinylierungsverfahren zur Darstellung von Produktvinylestern, deren Siedepunkte höher liegen als die der Eduktvinylester. Durch Reaktivdestillation von mindestens einer der Produktkomponenten wird das Gleichgewicht der Reaktion auf die Produktseite verschoben. Der zugleich destillierte Eduktvinylester wird der Reaktion rückgeführt. Bei Verwendung von Ru-Katalysatoren wird die Bildung von Anhydriden als Nebenprodukt beschrieben. Begünstigt wird deren Bildung durch hohe Reaktionstemperatur, hohen Umsatzgrad, lange Verweilzeit sowie durch eine hohe Konzentration der Reaktanden. Die Autoren empfehlen für eine maximale Selektivität bzw. die Minimierung der Anhydrid-Bildung die Reaktion bei möglichst niedrigem Umsatzgrad und geringer Verweilzeit durchzuführen. Der Wiedereinsatz eines Ru-haltigen Katalysators unter solchen Bedingungen wird in Beispiel 3 beschrieben. Hier erfolgt die Umvinylierung von Neodecansäure bei einem Umsatz von nur 50 %. Die Reaktivdestillation bedingt jedoch hohe Überschüsse an Eduktvinylester, die aufgrund der kurzen Verweilzeit nötig sind, um ausreichend Reaktionspartner für die Eduktcarbonsäure bereitzustellen. Hohe Raum-Zeit-Ausbeuten sind mit diesem Verfahren somit nicht erreichbar.

In der WO 92/09554 A1 wird ein Verfahren beschrieben, bei dem die Reaktionsmasse nach der Umvinylierung in einem ersten Schritt von Eduktcarbonsäure und Katalysator separiert und der Produktvinylester anschließend durch Azeotropdestillation abgetrennt wird. Dieses Verfahren zielt vor allem auf die Trennung von Säure/Vinylester-Gemischen mit geringen Siedepunktdifferenzen. Die Umvinylierung selbst wird kontinuierlich betrieben. Nur Eduktvinylester sowie ein Gemisch aus Katalysator und Eduktcarbonsäure werden dem Reaktor kontinuierlich rückgeführt. Die Bildung von Anhydriden sowie deren Rückführung wird nicht beschrieben.

Wegen der bereits beschriebenen Problematik beim Einsatz von Ru-Katalysator, der geringen Selektivität aufgrund Anhydridbildung, wird in den jüngeren Publikationen des Stands der Technik in den konkret offenbarten Ausführungsformen überwiegend mit Palladium-Katalysatoren gearbeitet. Die WO 2011/139360 A1 beschreibt ein kontinuierliches Verfahren zur Darstellung von Carbonsäurevinylestern durch Reaktivdestillation. Dabei werden Vinylacetat als Eduktvinylester und die daraus entstehende Essigsäure kontinuierlich abdestilliert, wobei das Vinylacetat dem Prozess rückgeführt wird. In den Beispielen werden ausschließlich Pd-katalysierte Systeme angeführt, welche sich durch eine hohe Selektivität auszeichnen und keine Anhydride bilden. Die WO 2011/139361 A1 beschreibt ein sehr ähnliches Verfahren, mit dem einzigen Unterschied, dass die Umvinylierung nicht kontinuierlich, sondern semi-kontinuierlich durchgeführt wird.

Die WO 2013/117294 A1 beschreibt ein kontinuierliches Verfahren zur Darstellung von Carbonsäurevinylestern. Im Unterschied zu den gerade diskutierten Reaktivdestillationsverfahren wird die übergangsmetallkatalysierte Umvinylierung im stationären Zustand betrieben und das Reaktionsgemisch in einem Folgeschritt aufgetrennt. WO 2013/117295 beschreibt eine weitere Ausgestaltung dieses Verfahrens mit einer nachträglichen Derivatisierung der entstehenden, konjugaten Säure des Eduktvinylesters. In beiden Schriften wird auf die geringe Ausbeute bzw. Selektivität Ru-katalysierter Umvinylierungen und auf eine Umsatzbeschränkte Fahrweise zur Unterdrückung der Anhydridbildung hingewiesen. Im Gegensatz zu den Pd-katalysierten Systemen werden in den Ru-katalysierten Beispielen beider Schriften trotz geringen Umsätzen niedrige Selektivitäten und in der Folge niedrige Raum-Zeit-Ausbeuten beschrieben.

Die Verwendung von Ru-Katalysatoren in der Umvinylierungsreaktion birgt deutliche Vorteile gegenüber Pd-Katalysatoren hinsichtlich Löslichkeit, Flüchtigkeit, thermische Stabilität und thermisch induzierbarer Aktivität. Als wesentlicher Nachteil dieser Systeme wird im Stand der Technik die im Gegensatz zur Pd-Katalyse signifikant auftretende Bildung von Anhydriden beschrieben, die die Selektivität des Prozesses und damit die Möglichkeit des Wiedereinsatzes des Katalysators deutlich erniedrigt. Nicht zuletzt begünstigt auch eine destillative Aufarbeitung des Produktvinylesters die Bildung von Anhydriden, da dabei hohe Temperaturen, lange Verweilzeiten und hohe Konzentrationen im Destillationssumpf auftreten. Auch bei einer äquimolaren Zusammensetzung bzw. geringen Überschüssen einer der Ausgangskomponenten und daher niedrigen theoretisch erreichbaren Umsätzen lässt sich die Anhydrid-Bildung daher nicht vollständig vermeiden. Solche Zusammensetzungen wären jedoch insofern von Interesse, da dadurch die Massenströme minimiert und hohe Raum-Zeit-Ausbeuten erhalten werden könnten. Die beschriebenen Reaktivdestillationsverfahren bedingen einen deutlichen Überschuss an Eduktvinylester, da dieser nach nur sehr kurzer Verweilzeit verdampft und für die Reaktion nicht mehr zur Verfügung steht. Hohe Raum-Zeit-Ausbeuten sind mit solchen Verfahren daher nicht erreichbar.

Es bestand daher die Aufgabe, ein Verfahren zur Umvinylierung zu entwickeln, welches sich durch eine hohe Selektivität bei gleichzeitig hoher Raum-Zeit-Ausbeute auszeichnet.

Gegenstand der Erfindung ist ein Verfahren zur selektiven Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren, wobei
a) der Eduktvinylester, die Eduktcarbonsäure und der Ruthenium-katalysator einem Reaktor zugeführt werden, dadurch gekennzeichnet, dass
b) das molare Verhältnis von Eduktvinylester zu Eduktcarbonsäure 1:3 bis 3:1 beträgt, und
c) die Umvinylierungsreaktion durchgeführt wird,
d) nach Abschluss der Umvinylierungsreaktion der Eduktvinylester und die korrespondierende Säure aus dem Reaktionsgemisch destillativ abgetrennt werden,
e) aus dem Sumpfprodukt der Destillation der Produktvinylester destillativ abgetrennt wird, und
f) das verbleibende Reaktionsgemisch in den Reaktor zurückgeführt wird.

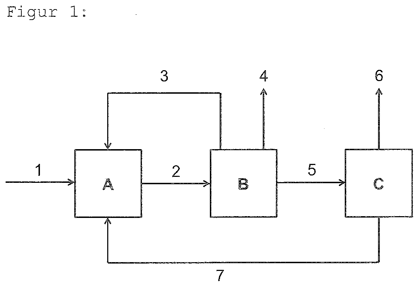

Der prinzipielle Ablauf des erfindungsgemäßen Verfahrens ist in Figur 1 dargestellt. Die Edukte (1) werden einem Reaktor (A) einzeln oder als Gemisch zugeführt. Im Reaktor (A) erfolgt die Umvinylierungsreaktion. Das resultierende Reaktionsgemisch (2) wird in einer Destillationsvorrichtung (B) von Eduktvinylester (3) und dessen korrespondierender Säure (4) befreit. Der Eduktvinylester (3) wird gegebenenfalls dem Reaktor (A) zurückgeführt. Vom verbleibenden Produktgemisch (5) wird anschließend der Produktvinylester (6) in einer Destillationsvorrichtung (C) vollständig oder teilweise abgetrennt. Der zurückbleibende Katalysatorhaltige Reaktionssumpf (7) wird in den Reaktor (A) zurückgeführt und der Katalysator somit erneut eingesetzt.

Als Reaktor (A) können Rührkessel, Rührkessel-Kaskaden oder Rohrreaktoren eingesetzt werden. Vorzugsweise ist der Reaktor (A) ein Rohrreaktor.

Als Eduktvinylester können beliebige Carbonsäurevinylester der allgemeinen Formel R-C(O)O-CH=CH₂ eingesetzt werden, wobei R ein aliphatischen Rest mit 1 bis 12 C-Atomen sein kann, oder ein cycloaliphatischer Rest mit bis zu 12 C-Atomen sein kann, oder ein aromatischer Rest mit bis zu 12 C-Atomen sein kann. Bevorzugt ist die Verwendung niedermolekularer Eduktvinylester, wobei R ein Alkylrest mit 1 bis 6 C-Atomen ist. Besonders bevorzugt ist die Verwendung von Vinylacetat.

Ferner wird dem Reaktor mindestens eine Eduktcarbonsäure der allgemeinen Formel R'-COOH zugeführt, wobei R' ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann. Bevorzugt werden Eduktcarbonsäuren der genannten Verbindungsklassen mit 6 bis 18 C-Atomen eingesetzt. Beispiele hierfür sind Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphtalincarbonsäure. Besonders bevorzugt werden Versaticsäuren^{R} (alphaverzweigte Carbonsäuren mit 9 bis 12 C-Atomen der Fa. Momentive) oder Neo-Säuren mit 9 bis 12 C-Atomen und Fettsäuren wie Laurinsäure, Myristinsäure, Palmitinsäure, Stearinsäure.

Als Katalysator geeignete Rutheniumverbindungen sind dem Fachmann bekannt, beispielsweise aus dem US-Patent 4,981,973 auf dessen diesbezügliche Offenbarung Bezug genommen wird. Geeignete Rutheniumverbindungen sind beispielsweise Rutheniumcarbonylverbindungen und Rutheniumcarboxylatverbindungen. Weitere Rutheniumverbindungen welche zu hoch aktiven Katalysatoren führen, sind Ruthenium(III)acetylacetonat, Ruthenium(IV)oxid, Ruthenium auf Träger wie Kohle oder Aluminiumoxid, Rutheniumhalogenide wie Ru(III)Chlorid und Ru(III)Jodid.

Der Ru-Katalysator wird typischerweise in Konzentrationen von 0,1 bis 10000 ppm (Gehalt Ruthenium bezogen auf die Reaktionsmasse aus Eduktvinylester und Eduktcarbonsäure) eingesetzt, bevorzugt ist die Verwendung von 1 bis 1000 ppm (Gehalt Ruthenium bezogen auf die Reaktionsmasse aus Eduktvinylester und Eduktcarbonsäure).

Den Edukten kann gegebenenfalls ein Polymerisationsinhibitor zugesetzt werden. Bevorzugt werden 100 bis 10000 ppm, bezogen auf die Reaktionsmasse aus Eduktvinylester und Eduktcarbonsäure, Polymerisationsinhibitor eingesetzt. Beispiele für Polymerisationsinhibitoren sind Hydrochinon, Methoxyhydrochinon, tertiär-Butylcatechol, Phenothiazin oder Nitroxidradikale wie TEMPO oder 4-OH-TEMPO (TEMPO = 2,2,6,6-Tetramethylpiperidinyloxyl). Bevorzugt ist die Verwendung von Phenothiazin oder Hydrochinon.

Gegebenenfalls kann auch ein Anhydrid der jeweiligen Eduktcarbonsäure als Edukt zugegeben werden. Die gegebenenfalls zugeführten Anhydride der Eduktcarbonsäure der allgemeinen Formel R¹-C(O)-O-C(O)-R² können als gemischte (R¹≠R²) oder symmetrische (R¹=R²) Anhydride vorliegen, wobei R¹ und R² jeweils einen aliphatischen Rest mit 1 bis 22 C-Atomen, oder einen cycloaliphatischen Rest mit bis zu 22 C-Atomen oder einen aromatischen Rest mit bis zu 22 C-Atomen darstellt. Beispiele hierfür sind gemischte oder symmetrische Anhydride folgender Säuren: Essigsäure, Propionsäure, n-Buttersäure, iso-Buttersäure, n-Valeriansäure, 2-Methylbuttersäure, 3-Methylbuttersäure, Pi-valinsäure, Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphtalincarbonsäure. Bevorzugt werden die symmetrischen Anhydride der Eduktcarbonsäure eingesetzt.

Zur Umvinylierung können die Reaktanden Eduktvinylester, Eduktcarbonsäure und Ru-Katalysator, sowie gegebenenfalls Inhibitor sowie gegebenenfalls Anhydrid der Eduktcarbonsäure, dem Reaktor einzeln oder in einem Gemisch zugeführt werden.

Es wurde gefunden, dass das molare Verhältnis von Eduktvinylester zu Eduktcarbonsäure die maximal erreichbare Raum-Zeit-Ausbeute bestimmt, da dadurch sowohl der theoretische erreichbare Umsatz der Gleichgewichtsreaktion als auch die Massenströme pro Volumeneinheit festgelegt sind. Hohe Raum-Zeit-Ausbeuten können somit nur mit einem äquimolaren Verhältnis von Eduktvinylester zu Eduktcarbonsäure oder geringen Überschüssen einer der beiden Ausgangskomponenten erreicht werden. Das molare Verhältnis von Eduktvinylester zu Eduktcarbonsäure kann 1 : 3 bis 3 : 1 betragen. Bevorzugt ist ein Verhältnis von Eduktvinylester zu Eduktcarbonsäure von 1 : 1 bis 2 : 1, besonders bevorzugt ist ein Verhältnis von etwa 1 : 1.

Die Umvinylierung wird im Allgemeinen bei einer Temperatur von 100°C bis 170°C, vorzugsweise bei einer Temperatur von 120°C bis 150°C durchgeführt. Der Druck, bei welchem die Umvinylierung erfolgt, ist von der Temperatur abhängig und beträgt im Allgemeinen ≥ 2 bar abs., vorzugsweise 5 bis 15 bar abs., und am meisten bevorzugt 5 bis 10 bar abs.. Die Reaktion wird bevorzugt in einer Schutzgasatmosphäre, beispielsweise Stickstoff, in an sich bekannter Weise, durchgeführt.

Die Verweilzeit im Reaktor beträgt bei dem erfindungsgemäßen Verfahren im Allgemeinen 0,25 bis 5 Stunden, vorzugsweise 1 Stunde bis 4 Stunden.

Im Unterschied zu einer Reaktivdestillation erfolgt im erfindungsgemäßen Verfahren die Auftrennung des erhaltenen Produktgemisches erst nach Abschluss der Umvinylierung vorzugsweise mittels Destillation in entsprechenden Destillationskolonnen. Druck und Temperatur der Destillation sowie die Auslegung der Destillationskolonnen hängen von den im Produktgemisch vorliegenden Komponenten ab und können beispielsweise mittels Routineversuchen vom Fachmann ermittelt werden. Bei dem erfindungsgemäßen Verfahren wird keine Azeotropdestillation durchgeführt. Bei der Auftrennung des Produktgemisches werden in einem ersten Schritt der nicht umgesetzte Rest des Eduktvinylesters und dessen korrespondierende Säure jeweils aus dem Produktgemisch abgetrennt. Der damit erhaltene Eduktvinylester kann gegebenenfalls zur neuerlichen Umvinylierung in den Reaktor zurückgeführt werden. Die damit erhaltene korrespondierende Säure des Eduktvinylesters kann als Edukt in anderen chemischen Prozessen eingesetzt werden; im Falle von Essigsäure beispielsweise zur Herstellung von Vinylacetat.

Zur weiteren Aufarbeitung des danach verbleibenden Produktgemisches können verschiedene Wege eingeschlagen werden:
In einer ersten Ausführungsform wird der Produktvinylester aus dem verbleibenden Produktgemisch durch Destillation zumindest teilweise oder vollständig abgetrennt. Der resultierende Reaktionssumpf, welcher Eduktcarbonsäure, Anhydride der Eduktcarbonsäure, Ruthenium-Katalysator und gegebenenfalls weitere Komponenten wie Produktvinylester oder polymere Bestandteile enthalten kann, wird unter Zusatz von frischen Edukten und gegebenenfalls frischem Katalysator in den Reaktor für eine neuerliche Umvinylierung zurückgeführt.

In einer alternativen Ausführungsform wird ebenfalls der Produktvinylester aus dem, nach Abtrennung des Eduktvinylesters und dessen korrespondierende Säure, verbleibenden Produktgemisch durch Destillation zumindest teilweise oder vollständig abgetrennt. Nach Abtrennung des Produktvinylesters wird dem resultierenden Reaktionssumpf Wasser zugegeben. Die zugegebene Wassermenge beträgt vorzugsweise 1 bis 5 Mol-Äquivalente bezogen auf die im Reaktionssumpf vorliegende Menge an Anhydrid der Eduktcarbonsäure. Besonders bevorzugt wird etwa ein Äquivalent Wasser pro Äquivalent Anhydrid der Eduktcarbonsäure zugegeben. Anschließend wird der Reaktionssumpf so behandelt, dass das Anhydrid der Eduktcarbonsäure vollständig hydrolysiert wird, vorzugsweise zu mindestens 80 Mol-%. Dazu wird eine Temperatur von 40°C bis 160°C, vorzugsweise von 100°C bis 140°C eingestellt. Die Hydrolyse wird über einen Zeitraum von 1 bis 30 Minuten, vorzugsweise 1 bis 10 Minuten ausgeführt. Der nach der Hydrolyse des Anhydrids der Eduktcarbonsäure erhaltene Reaktionssumpf, welcher Eduktcarbonsäure, Ru-Katalysator und gegebenenfalls weitere Komponenten wie Produktvinylester oder polymere Bestandteile enthalten kann, wird unter Zusatz von frischen Edukten und gegebenenfalls frischem Katalysator in den Reaktor für eine neuerliche Umvinylierung zurückgeführt.

In einer weiteren Ausführungsform wird dem, nach Abtrennung des Eduktvinylesters und dessen korrespondierende Säure, verbleibenden Produktgemisch in einem ersten Schritt Wasser zugegeben. Die zugegebene Wassermenge beträgt vorzugsweise 1 bis 5 Mol-Äquivalente bezogen auf die im Reaktionssumpf vorliegende Menge an Anhydrid der Eduktcarbonsäure. Besonders bevorzugt wird etwa ein Äquivalent Wasser pro Anhydrid der Eduktcarbonsäure zugegeben. Anschließend wird der Reaktionssumpf so behandelt, dass das Anhydrid der Eduktcarbonsäure vollständig hydrolysiert wird, vorzugsweise zu mindestens 80 Mol-%. Dazu wird eine Temperatur von 40°C bis 160°C, vorzugsweise von 100°C bis 140°C eingestellt. Die Hydrolyse wird über einen Zeitraum von 1 bis 30 Minuten, vorzugsweise 1 bis 10 Minuten ausgeführt. Anschließend wird der Produktvinylester zumindest teilweise oder vollständig mittels Destillation abgetrennt. Der nach der Destillation des Produktvinylesters erhaltene Reaktionssumpf, welcher Eduktcarbonsäure, Ru-Katalysator und gegebenenfalls weitere Komponenten wie Produktvinylester oder polymere Bestandteile enthalten kann, wird unter Zusatz von frischen Edukten und gegebenenfalls frischem Katalysator in den Reaktor für eine neuerliche Umvinylierung zurückgeführt.

Der Zusatz von frischen Edukten und gegebenenfalls frischem Katalysator in den Reaktor für eine neuerliche Umvinylierung kann jeweils im Gemisch mit dem zurückgeführten Reaktionssumpf erfolgen oder jeweils getrennt vom zurückgeführten Reaktionssumpf erfolgen.

Im Produktgemisch vorliegende polymere Bestandteile können gegebenenfalls teilweise oder vollständig abgetrennt werden. Die Abtrennung der polymeren Bestandteile kann beispielsweise über Filtration, Extraktion, Sedimentation oder Fällung erfolgen, bevorzugt ist die Abtrennung über Fällung, Sedimentation oder Filtration. Die Abtrennung kann vor oder erst nach einem Wasserzusatz erfolgen. Die Abtrennung erfolgt unter Rückhaltung des Ru-Katalysators, sodass die abgetrennten polymeren Bestandteile eine geringere Masse an Ruthenium aufweist, als der in den Reaktor (A) zurückgeführte Reaktionssumpf (7).

Die Teilschritte des Verfahrens, sowohl die Umvinylierung als auch die Aufarbeitungsschritte, können diskontinuierlich, semi-kontinuierlich und vollkontinuierlich durchgeführt werden. Das Verfahren wird bevorzugt vollkontinuierlich durchgeführt.

Mit dem erfindungsgemäßen Verfahren können auch bei hohen Raum-Zeit-Ausbeuten von vorzugsweise ≥ 280 g/l*h, besonders bevorzugt ≥ 300 g/l*h, am meisten bevorzugt ≥ 330 g/l*h, hohe Selektivitäten erreicht werden. Die Raum-Zeit-Ausbeute berechnet sich nach RZA (g/l*h) = m_{P}/ (V_{R}*t_{R}) , wobei m_{P} die Masse an Produktvinylester, V_{R} das Eduktgesamtvolumen und t_{R} die Reaktionszeit darstellt. Die Selektivität der Umvinylierungsreaktion beträgt vorzugsweise ≥ 95 %, besonders bevorzugt ≥ 97 %, am meisten bevorzugt ≥ 99 %. Die Selektivität bezieht sich auf die Ausbeute A an Produktvinylester pro umgesetzter Ausgangskomponente, gemäß S = A/U. Die Ausbeute berechnet sich nach A (%) = 100 x (p_{E}-p₀)/n₀, wobei p_{E} die Stoffmenge des nach der Reaktion erhaltenen Produktvinylesters darstellt, p₀ die Stoffmenge des Produktvinylesters zu Beginn der Reaktion (im wieder eingesetzten katalysatorhaltigen Reaktionssumpf enthalten) und n₀ die Stoffmenge der im geringeren molaren Anteil eingesetzten Ausgangskomponente darstellt.

Eine Akkumulation von Carbonsäureanhydrid durch wiederholte Rückführung von Katalysator und Anhydride der Eduktcarbonsäure enthaltendem Reaktionssumpf, wurde überraschenderweise nicht beobachtet. Überraschend wurde gefunden, dass Carbonsäureanhydride in der Ru-katalysierten Umvinylierungsreaktion zu Produktvinylestern umgesetzt werden können.

Ferner kann durch Zugabe von Wasser das Carbonsäureanhydrid hydrolysiert werden. Überraschend wurde gefunden, dass auch durch Zugabe größerer Mengen Wasser zum Ru-haltigen Reaktionssumpf die Aktivität des Katalysators beim Wiedereinsatz zur Umvinylierung nicht beeinträchtigt wird.

Das erfindungsgemäße Verfahren ermöglicht somit eine wirtschaftliche Fahrweise der Umvinylierung, bei der der Ru-Katalysator durch Rückführung des Reaktionssumpfes wiederholt eingesetzt werden kann.

### Beispiele:

Die nachfolgenden Beispiele dienen der näheren Erläuterung der Erfindung.

Die Ausbeute berechnet sich nach A (%) = 100 x (p_{E}-p₀)/n₀, wobei p_{E} die Stoffmenge des nach der Reaktion erhaltenen Produktvinylesters darstellt, p₀ die Stoffmenge des Produktvinylesters zu Beginn der Reaktion (im wiedereingesetzten katalysatorhaltigen Reaktionssumpf enthalten) und n₀ die Stoffmenge der im geringeren molaren Anteil eingesetzten Ausgangskomponente darstellt.

Die Raum-Zeit-Ausbeute berechnet sich nach RZA (g/l*h) = m_{P}/(V_{R}*t_{R}), wobei m_{P} die Masse an Produktvinylester, V_{R} das Eduktgesamtvolumen und t_{R} die Reaktionszeit darstellt.

Angegebene Selektivitäten S beziehen sich auf die Ausbeute A an Produktvinylester pro umgesetzter Ausgangskomponente, gemäß S = A/U. Der Umsatz U ist definiert als U (%) = 100 x (n₀-n_{E})/n₀, wobei n₀ die Stoffmenge der Ausgangskomponente zu Beginn der Reaktion und n_{E} die Stoffmenge am Ende der Reaktion darstellt.

### Vergleichsbeispiel 1:

Umvinylierung bei niedriger Raum-Zeit-Ausbeute (molares Verhältnis Laurinsäure/Vinylacetat = 1:6) und hoher Selektivität mit Rückführung des Anhydrid-haltigen Reaktionssumpfes.

In einem 100 ml Berghoff-Autoklav wurden 20,5 g Laurinsäure, 52,9 g Vinylacetat und 0,016 g Trirutheniumdodecacarbonyl bei 5 bar abs. 1 Stunde lang auf 140°C erhitzt. Nach dem Abkühlen wurden Essigsäure und Vinylacetat am Rotationsdampfer entfernt und Vinyllaurat im Vakuum destilliert. Der Rückstand aus Laurinsäureanhydrid, Laurinsäure und Ru-Katalysator wurde nach Zusatz von frischer Laurinsäure und frischem Vinylacetat unter gleichen Bedingungen erneut eingesetzt.

Bei jedem Umlauf wurden die für die Berechnung von Raum-Zeit-Ausbeute und Selektivität benötigten Mengen und molaren Anteile im Reaktionsgemisch mittels quantitativer NMR-Spektroskopie ermittelt.

| **Umlauf** | | **Molare Anteile** | | | **Raum-Zeit-Ausbeute** | **Selektivität** |
|---|---|---|---|---|---|---|
| | | **Laurinsäure** | **Vinyllaurat** | **Laurinsäureanhydrid** | **Vinyllaurat [g/(l*h)]** | **Vinyllaurat [%]** |
| **1** | Start | 0,990 | 0,010 | 0,000 | | |
| | Ende | 0,139 | 0,854 | 0,008 | 247 | 99,1 |
| **2** | Start | 0,961 | 0,022 | 0,018 | | |
| | Ende | 0,136 | 0,853 | 0,012 | 250 | 100,7 |
| **3** | Start | 0,965 | 0,010 | 0,025 | | |
| | Ende | 0,160 | 0,830 | 0,010 | 246 | 101,9 |
| **4** | Start | 0,968 | 0,010 | 0,022 | | |
| | Ende | 0,126 | 0,859 | 0,015 | 254 | 100,8 |
| **5** | Start | 0,957 | 0,014 | 0,029 | | |
| | Ende | 0,146 | 0,840 | 0,014 | 250 | 101,9 |

Das Beispiel zeigt, dass es beim Wiedereinsatz von katalysatorhaltigem Reaktionssumpf nach einem ersten Anstieg zu keiner weiteren Akkumulation von Laurinsäureanhydrid kommt. Ferner erkennt man, dass durch die destillative Aufarbeitung, die zwischen dem Ende der Reaktion eines Umlaufes und dem Start des nächsten Umlaufes liegt, der Anhydridanteil ansteigt. Ein Teil dieses Anhydrids wird im folgenden Umlauf auch zu Vinyllaurat umgesetzt, woraus rechnerisch Selektivitäten größer 100 % erhalten werden. Ferner wird deutlich, dass bei einem eingesetzten molaren Laurinsäure/Vinylacetat-Verhältnis von 1 : 6 lediglich Raum-Zeit-Ausbeuten < 260 g/l*h erhalten werden.

### Beispiel 2:

Umvinylierung bei hoher Raum-Zeit-Ausbeute (molares Verhältnis Laurinsäure/Vinylacetat = 1:1) und hoher Selektivität mit Rückführung des Anhydrid-haltigen Reaktionssumpfes.

In einem 100 ml Berghoff-Autoklav wurden 50 g Laurinsäure, 21,5 g Vinylacetat und 0,16 g Trirutheniumdodecacarbonyl bei 2 bar abs. 1 Stunde lang auf 140°C erhitzt. Nach dem Abkühlen wurden Essigsäure und Vinylacetat am Rotationsdampfer entfernt und Vinyllaurat im Vakuum destilliert. Der Rückstand aus Laurinsäureanhydrid, Laurinsäure und Ru-Katalysator wurde nach Zusatz von frischer Laurinsäure und frischem Vinylacetat unter gleichen Bedingungen erneut eingesetzt.

| **Umlauf** | | **Molare Anteile** | | | **Raum-Zeit-Ausbeute** | **Selektivität** |
|---|---|---|---|---|---|---|
| | | **Laurinsäure** | **Vinyllaurat** | **Laurinsäureanhydrid** | **Vinyllaurat [g/(l*h)]** | **Vinyllaurat [%]** |
| **1** | Start | 0,995 | 0,005 | 0,000 | | |
| | Ende | 0,488 | 0,493 | 0,019 | 348 | 96,3 |
| **2** | Start | 0,994 | 0,005 | 0,001 | | |
| | Ende | 0,580 | 0,400 | 0,020 | 282 | 95,3 |
| **3** | Start | 0,963 | 0,005 | 0,033 | | |
| | Ende | 0,558 | 0,419 | 0,022 | 305 | 102,6 |
| **4** | Start | 0,959 | 0,006 | 0,035 | | |
| | Ende | 0,501 | 0,472 | 0,027 | 344 | 101,8 |
| **5** | Start | 0,926 | 0,035 | 0,040 | | |
| | Ende | 0,496 | 0,475 | 0,030 | 337 | 102,3 |

Das Beispiel zeigt, dass bei Einsatz eines Laurinsäure/Vinylacetat-Verhältnis von 1:1 deutlich höhere Raum-Zeit-Ausbeuten (> 280 g/l*h) erhalten werden. Gleichzeitig wird neben der eingesetzten Laurinsäure ein Anteil des Anhydrids zu Vinyllaurat umgesetzt. Anhydrid akkumuliert nicht.

### Beispiel 3:

### Umvinylierung von Laurinsäureanhydrid

In einem 100 ml Berghoff-Autoklav wurden 25 g Laurinsäureanhydrid und 454 ppm Ru in Form eines katalysatorhaltigen Reaktionssumpfes vorgelegt und bei 6 bar abs. auf 140°C erhitzt. Bei dieser Temperatur erfolgte die Zugabe von 45 g Vinylacetat. Anschließend wurde die Zusammensetzung der Reaktionsmischung im zeitlichen Verlauf NMR-spektroskopisch untersucht.

| **Zeit** | **Umsatz [%]** | **Ausbeute [%] Vinyllaurat** | **Selektivität [%]** |
|---|---|---|---|
| **10min** | 18,5 | 11,4 | 62 |
| **20min** | 27,4 | 21,1 | 77 |
| **30min** | 34,4 | 28,7 | 83 |
| **45min** | 40,1 | 35,1 | 88 |
| **60min** | 45,7 | 41,9 | 92 |
| **90min** | 54,0 | 50,5 | 94 |
| **120min** | 60,2 | 57,2 | 95 |

Das Beispiel demonstriert, dass auch Laurinsäureanhydrid unter den gegebenen Bedingungen zu Vinyllaurat umgesetzt wurde. Im stationären Zustand liegt auch hier der Umsatz höher als 60 %.

### Beispiel 4:

Umvinylierung bei hoher Raum-Zeit-Ausbeute (molares Verhältnis Laurinsäure/Vinylacetat = 1:3) und hoher Selektivität mit Rückführung des Anhydrid-haltigen Reaktionssumpfes.

In einem 100 ml Berghoff-Autoklav wurden 23,2 g Laurinsäure, 29,9 g Vinylacetat und 0,074 g Trirutheniumdodecacarbonyl bei 4 bar abs. 1 Stunde lang auf 140°C erhitzt. Nach dem Abkühlen wurden Essigsäure und Vinylacetat am Rotationsdampfer entfernt und Vinyllaurat im Vakuum destilliert. Der Rückstand aus Laurinsäureanhydrid, Laurinsäure und Ru-Katalysator wurde nach Zusatz von frischer Laurinsäure und frischem Vinylacetat unter gleichen Bedingungen erneut eingesetzt.

| **Umlauf** | | **Molare Anteile** | | | **Raum-Zeit-Ausbeute** | **Selektivität** |
|---|---|---|---|---|---|---|
| | | **Laurinsäure** | **Vinyllaurat** | **Laurinsäureanhydrid** | **Vinyllaurat [g/(l*h)]** | **Vinyllaurat [%]** |
| **1** | Start | 0,996 | 0,004 | 0,000 | | |
| | Ende | 0,253 | 0,741 | 0,006 | 331 | 99,2 |
| **2** | Start | 0,932 | 0,053 | 0,015 | | |
| | Ende | 0,223 | 0,759 | 0,018 | 340 | 99,5 |
| **3** | Start | 0,899 | 0,023 | 0,078 | | |
| | Ende | 0,233 | 0,735 | 0,032 | 355 | 106,9 |
| **4** | Start | 0,910 | 0,008 | 0,083 | | |
| | Ende | 0,240 | 0,733 | 0,027 | 358 | 108,3 |
| **5** | Start | 0,913 | 0,028 | 0,058 | | |
| | Ende | 0,249 | 0,729 | 0,022 | 344 | 105,4 |

### Beispiel 5:

Umvinylierung bei hoher Raum-Zeit-Ausbeute (molares Verhältnis Laurinsäure/Vinylacetat = 1:3) und hoher Selektivität mit Rückführung des Reaktionssumpfes nach Wasserzugabe (Vinyllauratabtrennung vor Wasserzugabe)

In einem 100 ml Berghoff-Autoklav wurden 32,5 g Laurinsäure, 41,9 g Vinylacetat und 0,010 g Trirutheniumdodecacarbonyl bei 4 bar abs. 1 Stunde lang auf 140°C erhitzt. Nach dem Abkühlen wurden Essigsäure und Vinylacetat am Rotationsdampfer entfernt und anschließend Vinyllaurat im Vakuum abdestilliert. Nach Zugabe von 5 Gew.-% Wasser (bezogen auf die Masse des Reaktionssumpfes) und nach 5 min Rühren bei 140°C wurde der Rückstand aus Laurinsäure und Ru-Katalysator nach Zusatz von frischer Laurinsäure und frischem Vinylacetat unter gleichen Bedingungen erneut zur Umvinylierung eingesetzt.

| **Umlauf** | | **Molare Anteile** | | | **Raum-Zeit-Ausbeute** | **Selektivität** |
|---|---|---|---|---|---|---|
| | | **Laurinsäure** | **Vinyllaurat** | **Laurinsäureanhydrid** | **Vinyllaurat [g/(l*h)]** | **Vinyllaurat [%]** |
| **1** | Start | 0,997 | 0,003 | 0,000 | | |
| | Ende | 0,226 | 0,766 | 0,008 | 343 | 99,0 |
| **2** | Start | 0,988 | 0,012 | 0,000 | | |
| | Ende | 0,221 | 0,757 | 0,021 | 338 | 97,2 |
| **3** | Start | 0,920 | 0,043 | 0,037 | | |
| | Ende | 0,217 | 0,765 | 0,018 | 352 | 102,8 |
| **4** | Start | 0,933 | 0,015 | 0,052 | | |
| | Ende | 0,210 | 0,762 | 0,028 | 359 | 103,4 |
| **5** | Start | 0,923 | 0,013 | 0,063 | | |
| | Ende | 0,212 | 0,761 | 0,026 | 363 | 105,2 |

### Beispiel 6:

Umvinylierung bei hoher Raum-Zeit-Ausbeute (molares Verhältnis Laurinsäure/Vinylacetat = 1:3) und hoher Selektivität mit Rückführung des Reaktionssumpfes nach Wasserzugabe (Vinyllauratabtrennung nach Wasserzugabe)

In einem 100 ml Berghoff-Autoklav wurden 26,9 g Laurinsäure, 34,7 g Vinylacetat und 0,086 g Trirutheniumdodecacarbonyl bei 4 bar abs. 1 Stunde lang auf 140°C erhitzt. Nach dem Abkühlen wurden Essigsäure und Vinylacetat am Rotationsdampfer entfernt. Nach Zugabe von 10 Gew.-% Wasser (bezogen auf das Produktgemisch) wurde Vinyllaurat im Vakuum abdestilliert. Der Rückstand aus Laurinsäure und Ru-Katalysator wurde nach Zusatz von frischer Laurinsäure und frischem Vinylacetat unter gleichen Bedingungen erneut zur Umvinylierung eingesetzt.

| **Umlauf** | | **Molare Anteile** | | | **Raum-Zeit-Ausbeute** | **Selektivität** |
|---|---|---|---|---|---|---|
| | | **Laurinsäure** | **Vinyllaurat** | **Laurinsäureanhydrid** | **Vinyllaurat [g/(l*h)]** | **Vinyllaurat [%]** |
| **1** | Start | 0,996 | 0,004 | 0,000 | | |
| | Ende | 0,253 | 0,741 | 0,006 | 331 | 99,2 |
| **2** | Start | 0,932 | 0,022 | 0,045 | | |
| | Ende | 0,263 | 0,720 | 0,018 | 336 | 104,1 |
| **3** | Start | 0,936 | 0,020 | 0,044 | | |
| | Ende | 0,276 | 0,708 | 0,016 | 330 | 104,2 |
| **4** | Start | 0,950 | 0,013 | 0,037 | | |
| | Ende | 0,271 | 0,716 | 0,013 | 332 | 103,5 |
| **5** | Start | 0,946 | 0,017 | 0,037 | | |
| | Ende | 0,261 | 0,724 | 0,015 | 336 | 103,2 |

Der Vergleich der Ergebnisse von Beispiel 4 mit denen von Beispiel 5 und 6 zeigt, dass es durch den Zusatz von Wasser (Beispiel 5 und 6) zu keiner Inhibierung der Ru-Katalyse nach Wiedereinsatz des Katalysators kommt. Es wurden ähnlich hohe Werte für die Raum-Zeit-Ausbeute und Selektivität erhalten.

### Beispiel 7:

### Abtrennung polymerer Bestandteile vom Reaktionssumpf

Aus 15,6 g eines Ru-haltigen (0,023g Ru) Reaktionssumpfes enthaltend Laurinsäure, Laurinsäureanhydrid, Vinyllaurat und polymeren Bestandteilen wurden 0,34 g polymere Bestandteile durch Sedimentation abgetrennt. Die verbliebene Lösung enthielt 0,021 g Ruthenium.
Das Beispiel zeigt, dass polymere Bestandteile ohne signifikante Verluste an Ru-Katalysator abgetrennt werden können.

## Patentansprüche

1. Verfahren zur selektiven Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren, wobei
a) der Eduktvinylester, die Eduktcarbonsäure und der Rutheniumkatalysator einem Reaktor zugeführt werden, **dadurch gekennzeichnet, dass**
b) das molare Verhältnis von Eduktvinylester zu Eduktcarbonsäure 1:3 bis 3:1 beträgt, und
c) die Umvinylierungsreaktion durchgeführt wird,
d) nach Abschluß der Umvinylierungsreaktion der Eduktvinylester und die korrespondierende Säure aus dem Reaktionsgemisch destillativ abgetrennt werden,
e) aus dem Sumpfprodukt der Destillation der Produktvinylester destillativ abgetrennt wird, und
f) das verbleibende Reaktionsgemisch in den Reaktor zurückgeführt wird.

2. Verfahren nach Anspruch 1, **dadurch gekennzeichnet, dass** nach der Abtrennung des Produktvinylesters in Schritt e) dem verbleibenden Reaktionssumpf Wasser zugegeben wird, und der Reaktionssumpf so behandelt wird, dass der Carbonsäureanhydridanteil hydrolysiert wird, und das danach verbleibende Reaktionsgemisch in den Reaktor zurückgeführt wird.

3. Verfahren nach Anspruch 1, **dadurch gekennzeichnet, dass** nach der Abtrennung von Eduktvinylester und der korrespondierende Säure aus dem Reaktionsgemisch in Schritt d), dem Sumpfprodukt der Destillation Wasser zugegeben wird, und der Reaktionssumpf so behandelt wird, dass der Carbonsäureanhydridanteil hydrolysiert wird, anschließend der Produktvinylester mittels Destillation abgetrennt wird, und das danach verbleibende Reaktionsgemisch in den Reaktor zurückgeführt wird.

4. Verfahren nach Anspruch 1 bis 3, **dadurch gekennzeichnet, dass** als Eduktvinylester ein Carbonsäurevinylester der allgemeinen Formel R-C(O)O-CH=CH₂ eingesetzt wird, wobei R ein aliphatischen Rest mit 1 bis 12 C-Atomen ist, oder ein cycloaliphatischer Rest mit bis zu 12 C-Atomen ist, oder ein aromatischer Rest mit bis zu 12 C-Atomen ist.

5. Verfahren nach Anspruch 1 bis 3, **dadurch gekennzeichnet, dass** als Eduktvinylester Vinylacetat eingesetzt wird.

6. Verfahren nach Anspruch 1 bis 5, **dadurch gekennzeichnet, dass** als Eduktcarbonsäure eine Carbonsäure der allgemeinen Formel R'-COOH eingesetzt wird, wobei R' ein aliphatischer Rest mit 1 bis 22 C-Atomen ist, oder ein cycloaliphatischer Rest mit bis zu 22 C-Atomen ist, oder ein aromatischer Rest mit bis zu 22 C-Atomen ist.

7. Verfahren nach Anspruch 1 bis 5, **dadurch gekennzeichnet, dass** als Eduktcarbonsäure eine Carbonsäure eingesetzt wird aus der Gruppe umfassend Versaticsäuren und Neo-Säuren, jeweils mit 9 bis 12 C-Atomen, und Fettsäuren wie Laurinsäure, Myristinsäure, Palmitinsäure, Stearinsäure.

8. Verfahren nach Anspruch 1 bis 7, **dadurch gekennzeichnet, dass** ein Anhydrid der jeweiligen Eduktcarbonsäure als Edukt zugegeben wird.

9. Verfahren nach Anspruch 1 bis 8, **dadurch gekennzeichnet, dass** im Reaktionsgemisch vorliegende polymere Bestandteile teilweise oder vollständig abgetrennt werden.

10. Verfahren nach Anspruch 1 bis 9, **dadurch gekennzeichnet, dass** die Teilschritte des Verfahrens jeweils vollkontinuierlich durchgeführt werden.

11. Verfahren nach Anspruch 1 bis 10, **dadurch gekennzeichnet, dass** die Selektivität der Umvinylierungsreaktion ≥ 95 % beträgt, bei einer Raum-Zeit-Ausbeute von ≥ 280 g/l*h.

## Claims

1. Process for selective transvinylation of a reactant carboxylic acid with a reactant vinyl ester to give a product vinyl ester and the corresponding acid of the reactant vinyl ester in the presence of one or more ruthenium catalysts,
wherein
a) the reactant vinyl ester, the reactant carboxylic acid and the ruthenium catalyst are supplied to a reactor, **characterized in that**
b) the molar ratio of reactant vinyl ester to reactant carboxylic acid is 1:3 to 3:1, and
c) the transvinylation reaction is conducted,
d) on completion of the transvinylation reaction, the reactant vinyl ester and the corresponding acid are removed from the reaction mixture by distillation,
e) the product vinyl ester is separated by distillation from the bottom product of the distillation, and
f) the remaining reaction mixture is recycled into the reactor.

2. Process according to Claim 1, **characterized in that**, after the separation of the product vinyl ester in step e), water is added to the remaining reaction bottoms, and the reaction bottoms is treated so that the carboxylic anhydride fraction is hydrolyzed, and the reaction mixture remaining thereafter is recycled into the reactor.

3. Process according to Claim 1, **characterized in that**, after the removal of reactant vinyl ester and the corresponding acid from the reaction mixture in step d), water is added to the bottom product of the distillation, and the reaction bottoms is treated so that the carboxylic anhydride fraction is hydrolyzed, the product vinyl ester is then separated by means of distillation, and the reaction mixture remaining thereafter is recycled into the reactor.

4. Process according to Claim 1 to 3, **characterized in that** a carboxylic vinyl ester of the general formula R-C(O)O-CH=CH₂ is used as reactant vinyl ester, where R is an aliphatic residue having 1 to 12 carbon atoms, or is a cycloaliphatic residue having up to 12 carbon atoms, or is an aromatic residue having up to 12 carbon atoms.

5. Process according to Claim 1 to 3, **characterized in that** vinyl acetate is used as reactant vinyl ester.

6. Process according to Claim 1 to 5, **characterized in that** a carboxylic acid of the general formula R'-COOH is used as reactant carboxylic acid, where R' is an aliphatic residue having 1 to 22 carbon atoms, or is a cycloaliphatic residue having up to 22 carbon atoms, or is an aromatic residue having up to 22 carbon atoms.

7. Process according to Claim 1 to 5, **characterized in that** the reactant carboxylic acid used is a carboxylic acid from the group comprising versatic acids and neo acids, in each case having 9 to 12 carbon atoms, and fatty acids such as lauric acid, myristic acid, palmitic acid and stearic acid.

8. Process according to Claim 1 to 7, **characterized in that** an anhydride of the respective reactant carboxylic acid is added as reactant.

9. Process according to Claim 1 to 8, **characterized in that** polymeric constituents present in the reaction mixture are partly or completely removed.

10. Process according to Claim 1 to 9, **characterized in that** the substeps of the process are each conducted non-stop.

11. Process according to Claim 1 to 10, **characterized in that** the selectivity of the transvinylation reaction is ≥ 95%, at a space-time yield of ≥ 280 g/l*h.

## Revendications

1. Procédé pour la transvinylation sélective d'un acide carboxylique de départ avec un ester de vinyle de départ en un ester de vinyle produit et en l'acide correspondant de l'ester de vinyle de départ, en présence d'un ou de plusieurs catalyseurs à base de ruthénium,
a) l'ester de vinyle de départ, l'acide carboxylique de départ et le catalyseur à base de ruthénium étant introduits dans un réacteur, **caractérisé en ce que**
b) le rapport molaire d'ester de vinyle de départ à acide carboxylique de départ vaut 1:3 à 3:1, et
c) la réaction de transvinylation est réalisée,
d) après la fin de la réaction de transvinylation, l'ester de vinyle de départ et l'acide correspondant sont séparés par distillation du mélange réactionnel,
e) l'ester de vinyle produit est séparé par distillation du produit du fond de la distillation et
f) le mélange réactionnel résiduel est recyclé dans le réacteur.

2. Procédé selon la revendication 1, **caractérisé en ce qu'**après la séparation de l'ester de vinyle produit dans l'étape e), de l'eau est ajoutée au fond de réaction qui reste et le fond de réaction est traité de manière telle que la proportion d'anhydride d'acide carboxylique est hydrolysée et le mélange réactionnel qui reste ensuite est recyclé dans le réacteur.

3. Procédé selon la revendication 1, **caractérisé en ce qu'**après la séparation de l'ester de vinyle de départ et de l'acide correspondant du mélange réactionnel dans l'étape d), de l'eau est ajoutée au produit de fond de la distillation et le fond de réaction est traité de manière telle que la proportion d'anhydride d'acide carboxylique est hydrolysée, l'ester de vinyle produit est ensuite séparé par distillation et le mélange réactionnel qui reste ensuite est recyclé dans le réacteur.

4. Procédé selon la revendication 1 à 3, **caractérisé en ce qu'**on utilise, comme ester de vinyle de départ, un ester vinylique d'acide carboxylique de formule générale R-C(O)O-CH=CH₂, R représentant un radical aliphatique comprenant 1 à 12 atomes de carbone ou un radical cycloaliphatique comprenant jusqu'à 12 atomes de carbone ou un radical aromatique comprenant jusqu'à 12 atomes de carbone.

5. Procédé selon la revendication 1 à 3, **caractérisé en ce qu'**on utilise, comme ester de vinyle de départ, de l'acétate de vinyle.

6. Procédé selon la revendication 1 à 5, **caractérisé en ce qu'**on utilise, comme acide carboxylique de départ, un acide carboxylique de formule générale R'-COOH, R' représentant un radical aliphatique comprenant 1 à 22 atomes de carbone ou un radical cycloaliphatique comprenant jusqu'à 22 atomes de carbone ou un radical aromatique comprenant jusqu'à 22 atomes de carbone.

7. Procédé selon la revendication 1 à 5, **caractérisé en ce qu'**on utilise, comme acide carboxylique de départ, un acide carboxylique du groupe comprenant les acides versatiques et les acides Neo, comprenant à chaque fois 9 à 12 atomes de carbone, et les acides gras, tels que l'acide laurique, l'acide myristique, l'acide palmitique, l'acide stéarique.

8. Procédé selon la revendication 1 à 7, **caractérisé en ce qu'**un anhydride de l'acide carboxylique de départ respectif est ajouté comme produit de départ.

9. Procédé selon la revendication 1 à 8, **caractérisé en ce que** des constituants polymères se trouvant dans le mélange réactionnel sont séparés partiellement ou complètement.

10. Procédé selon la revendication 1 à 9, **caractérisé en ce que** les étapes partielles du procédé sont à chaque fois réalisées de manière complètement continue.

11. Procédé selon la revendication 1 à 10, **caractérisé en ce que** la sélectivité de la réaction de transvinylation est ≥ 95%, à un rendement espace-temps ≥ 280 g/l*h.
